# EUROPEAN PATENT SPECIFICATION

(11) **EP 1 889 598 B1**
(45) Date of publication and mention of the grant of the patent: **03.02.2016**
(21) Application number: 07108278.8
(22) Date of filing: 15.05.2007
(51) Int. Cl.: A61K 8/25, A61K 8/26, A61K 8/27, A61K 8/29, A61Q 1/12, A61K 8/19

(54) **Cosmetic composition in powdered form**
Kosmetische Zusammensetzung in Pulverform
Composition cosmétique sous forme de poudre

(30) Priority: 18.07.2006 US 831445 P
(43) Date of publication of application: 20.02.2008
(73) Proprietor: L'Oréal, 75008 Paris (FR)
(72) Inventor: Geffroy, Nathalie, 91190 Gif sur Yvette (FR); Valois, Audrey, 91190, Villiers le Bacle (FR)
(74) Representative: Chantraine, Sylvie Hélène

(56) References cited:
- EP-A- 1 033 126
- EP-A- 1 559 394
- JP-A- 10 182 397
- US-B1- 6 395 301

## Description

The subject of the present invention is a cosmetic make-up composition for the skin in powdered form, comprising a high content of pigments. The subject of the invention is also a method for making up the skin comprising the application of the said composition to the skin.

The make-up composition according to the invention is in particular a make-up composition for the skin in powdered form, especially as a face or body powder, an eye shadow, a blusher or a concealer product. More especially, the invention relates to a face powder.
Make-up compositions for the skin are commonly used to give an aesthetic colour to the skin, in particular to the face, but also to conceal skin imperfections such as blotches and spots.

Some make-up compositions are provided in loose powder or compact powder form. These compositions generally comprise a high content of powders, in particular at least 80% by weight, relative to the total weight of the composition.

**The document** EP 1 033 126 **belonging to the powder field discloses examples of compact powder.**

Among the powders commonly used, zinc oxide or titanium dioxide are of interest for providing covering power (that is to say allowing good concealing of skin colour defects) and also make it possible to protect the skin from the ultraviolet rays of the sun.

However, when they are introduced into cosmetic formulas, these powders have the disadvantage of conferring onto the products a dry and rough feel during their application to the skin and thus hamper the obtaining of properties of softness.

In addition, when these powders are applied to the skin, the make-up obtained is often very opaque, visible and has a pronounced powdery appearance which is not close to the natural grain of the skin: such a make-up is not natural.

The compositions in powdered form are often liked by users having a skin with a tendency to be greasy, in particular for their texture. However, the make-up obtained by loose powders has a poor resistance over time to rubbing or to sebum.

The aim of the present invention is therefore to have available a make-up composition for the skin in powdered form having good spreading properties during its application to the skin and conferring a make-up capable of effectively concealing skin colour defects with no "mask" effect.

Indeed, when the compositions in powdered form exhibit good slip, the user can work it more easily when they apply it to the skin, and the make-up obtained is more homogeneous.

The inventors have shown that by combining specific particles in a composition in powdered form, it was possible to obtain a make-up which makes it possible to effectively conceal skin defects, in particular colour defects, while conferring a natural and satiny appearance on the made-up skin.

The compositions obtained also exhibit good properties of softness during their application to the skin.

According to a first aspect, the subject of the invention is a **anhydrous cosmetic composition in loose powdered form comprising at least one pulverulent phase and a fatty binder, the said pulverulent phase**
**i) 15 to 70% by weight, relative to the total weight of the composition, of at least one pigment, and,**
**ii) 30 to 85% by weight, relative to the total weight of the composition, of at least one lamellar particle other than the pigment, the pulverulent phase comprising at least one lamellar pearlescent agent and at least two lamellar fillers chosen from talc, mica, boron nitride, kaolin and a mixture thereof, wherein one of the lamellar fillers is boron nitride being present in the composition in an amount ranging from 3 to 10% by weight, relative to the total weight of the composition, preferably from 5 to 8% by weight.**

The subject of the invention is also a method for making up the skin of the face and/or of the body, comprising the application, to the said skin, of a composition as defined above.

The subject of the invention is also the use of a composition as described above for producing a make-up capable of concealing skin colour defects, while conferring a natural and satiny appearance on the made-up skin.

The composition according to the invention is provided in powdered form which may be in particular a loose powder or a compact powder (which can be disintegrated in the dry state or with water).

### Pulverulent phase

The composition according to the invention comprises a pulverulent phase comprising at least one pigment and at least one lamellar particle.

### Pigments

The expression pigments should be understood to mean particles of any shape, which are white or coloured, inorganic or organic, insoluble in the physiological medium and intended to colour the composition.
The pigments may be white and/or coloured, inorganic and/or organic.
According to a particular embodiment, the composition according to the invention may comprise at least one pigment chosen from inorganic pigments. These inorganic pigments may in particular be chosen from pigments of metal oxides.

There may be mentioned, among the inorganic pigments, titanium dioxide, which is optionally surface-treated, zirconium or cerium oxides and zinc, iron (black, yellow or red) or chromium oxides, manganese violet, ultramarine blue, chromium hydrate and ferric blue, metallic powders such as aluminium powder and copper powder.

According to a preferred embodiment, the inorganic pigments, in particular of metal oxides, present in the composition according to the invention are chosen from titanium dioxide, zinc oxide and/or iron oxide.

According to a particular embodiment, the pulverulent phase of the composition according to the invention may comprise at least two different pigments.

According to a further preferred embodiment, the pulverulent phase of the composition according to the invention may comprise at least a first pigment chosen from iron oxides and at least a second pigment chosen from zinc oxides and/or titanium dioxide.

The iron oxides may be present in the composition according to the invention in an amount ranging from 1 to 40% by weight, relative to the total weight of the composition, preferably from 3 to 30%, and more preferably from 4 to 25% by weight.

The zinc oxides may be present in the composition according to the invention in an amount ranging from 5 to 55% by weight, relative to the total weight of the composition, preferably from 10 to 40% by weight, and more preferably from 15 to 25% by weight.
The titanium dioxide may be present in the composition according to the invention in an amount ranging from 3 to 55% by weight, relative to the total weight of the composition, preferably from 10 to 40% by weight, and more preferably from 15 to 25% by weight.

According to a preferred embodiment, the zinc oxide and titanium dioxide pigments may be present in the composition according to the invention in an amount such that the zinc oxide to titanium dioxide weight ratio ranges from 0.5 to 2, preferably from 0.8 to 1.8, and more preferably still from 0.9 to 1.7.

According to a further preferred embodiment, the iron oxide and zinc oxide pigments and the titanium dioxide pigments may be present in the composition according to the invention in an amount such that the weight ratio of the sum of the zinc oxides and titanium dioxides to the iron oxides ranges from 0.1 to 10, preferably from 0.2 to 8.

In addition to the inorganic pigments, the pulverulent phase of the composition according to the invention may comprise organic pigments. Among the organic pigments, there may be mentioned carbon black, D & C type pigments, lacquers based on carmine, barium, strontium, calcium and aluminium.

The pulverulent phase may also comprise goniochromatic pigments; these pigments exhibit a relatively high change of colour with the angle of observation.

The goniochromatic pigment may be chosen for example from pigments with interferential multilayer structure and liquid crystal pigments.

In the case of a multilayer structure, the latter may comprise for example at least two layers, each layer, independently of the other layer (s) or not, being produced for example from at least one material chosen from the group consisting of the following materials: MgF₂, CeF₃, ZnS, ZnSe, Si, SiO₂, Ge, Te, Fe₂O₃, Pt, Va, Al₂O₃, MgO, Y₂O₃, S₂O₃, SiO, HfO₂, ZrO₂, CeO₂, Nb₂O₅, Ta₂O₅, TiO₂, Ag, Al, Au, Cu, Rb, Ti, Ta, W, Zn, MoS₂, cryolite, alloys, polymers and combinations thereof. The goniochromatic agents with multilayer structures are in particular those described in the following documents: US-A-3 438 796, EP-A-227423, US-A-5 135 812, EP-A-170439, EP-A-341002, US-A-4 930 866, US-A-5 641 719, EP-A-472371, EP-A-395420, EP-A-753545, EP-A-768343, EP-A-571836, EP-A-708154, EP-A-579091, US-A-5 411 586, US-A-5 364 467, WO-A-97/39066, DE-A-4 225 031, WO 9517479 (BASF), DE-A-196 14 637. They exist in the form of glitter with a metallic colour.

The multilayer structures which can be used in the invention are, for example, the following structures: Al/SiO₂/Al/SiO₂/Al; Cr/MgF₂/Al/MgF₂/Al; MoS₂/SiO₂/Al/SiO₂/MoS₂; Fe₂O₃/SiO₂/Al/SiO₂/Fe₂O₃; Fe₂O₃/SiO₂/Fe₂O₃/SiO₂/Fe₂O₃; MoS₂/SiO₂/mica-oxide/SiO₂/MoS₂; Fe₂O₃/SiO₂/mica-oxide/SiO₂/Fe₂O₃. Various colours are obtained according to the thickness of the various layers. Thus, with the Fe₂O₃/SiO₂/Al/SiO₂/Fe₂O₃ structure, the colour goes from golden-green to grey-red for SiO₂ layers from 320 to 350 nm; from red to golden for SiO₂ layers from 380 to 400 nm; from violet to green for SiO₂ layers from 410 to 420 nm; from copper to red for SiO₂ layers from 430 to 440 nm. Consequently, the multilayer structure may be mainly inorganic or organic. Various colours are obtained depending on the thickness of each of the various layers. I

The goniochromatic pigments with an interferential multilayer structure according to the invention are in particular those described in the following documents: US-A-3 438 796, EP-A-227423, US-A-5 135 812, EP-A-170439, EP-A-341002, US-A-4 930 866, US-A-5 641 719, EP-A-472371, EP-A-395410, EP-A-753545, EP-A-768343, EP-A-571836, EP-A-708154, EP-A-579091, US-A-5 411 586, US-A-5 364 467, WO-A-97/39066, DE-A-4 225 031, WO 9517479 (BASF), DE-A-196 14 637, and combinations thereof. They exist in the form of glitter with a metallic colour.

The goniochromatic pigment with an interferential multilayer structure may be chosen from the group consisting of the following commercial goniochromatic pigments: Infinite Colors from the company Sihiseido, Sicopearl Fantastico from BASF, Colorstream, Xirallic, Xirona from Merck, Colorglitter from FLEX, and mixtures thereof.

Liquid crystal pigments are in particular described in application EP-A-1046692.
As liquid crystal particles, it is possible to use in particular those known under the CTFA name polyacrylate-4 and sold under the names "HELICONE^{®} HC Sapphire", HELICONE^{®} HC Scarabeus", "HELICONE^{®} HC Jade", "HELICONE^{®} HC Maple", "HELICONE^{®} HC XL Sapphire", "HELICONE^{®} HC XL Scarabeus", "HELICONE^{®} HC XL Jade", "HELICONE^{®} HC XL Maple" by the company WACKER.
The pigments may be present in the composition according to the invention in an amount ranging from 15 to 70% by weight, relative to the total weight of the composition, preferably from 25 to 70% by weight, more preferably from 26 to 60% by weight, and more preferably still from 27 to 50% by weight.

### Lamellar particles

**The pulverulent phase of the composition of the invention comprises at least one lamellar pearlescent agent and at least two lamellar fillers chosen from talc, mica, boron nitride, kaolin and a mixture thereof, wherein one of the lamellar fillers is boron nitride being present in the composition in an amount ranging from 3 to 10% by weight, relative to the total weight of the composition, preferably from 5 to 8% by weight.**

The expression lamellar particle for the purposes of the present invention is understood to mean particles existing in the form of optionally stratified sheets.

These sheets are characterized by a thickness which is smaller than the largest dimension. Preferably, the ratio between the largest dimension and the thickness is between 2 and 100.

The lamellar particles present in the composition according to the invention may be chosen from lamellar pearlescent agents, lamellar fillers and/or lamellar reflecting particles.

The expression pearlescent agents should be understood to mean iridescent particles, in particular produced by certain molluscs in their shell or alternatively synthesized.

The lamellar pearlescent agents may be chosen from white pearlescent agents such as mica coated with titanium or with bismuth oxychloride, coloured pearlescent agents such as mica-titanium coated with iron oxides, mica-titanium coated with in particular ferric blue or chromium oxide, mica-titanium coated with an organic pigment of the abovementioned type and pearlescent agents made of bismuth oxychloride or based on bismuth oxychloride. According to a preferred embodiment, the lamellar pearlescent agent is a pearlescent agent made of bismuth oxychloride, in particular sold under the name BIRON LF 2000 by the company MERCK.

The lamellar pearlescent agents may be present in the composition according to the invention in an amount ranging from 3 to 70% by weight, relative to the total weight of the composition, preferably from 5 to 40% by weight.
The lamellar fillers are chosen from:
- talc which is a magnesium silicate hydrate, and in particular those marketed under the names "Talc Luzenac 00" by the company LUZENAC, "Talc P3" by the company NIPPON TALC;
- kaolin which is an aluminium silicate hydrate which exists in the form of particles with an anistropic shape having sizes generally less than 30 µm; as kaolin, it is possible to use the one sold under the name "Kaolin Supreme 1" from ENGLISH CHINA LAYS;
- boron nitride, and in particular those marketed under the names "Ceram Blanche 1", "Ceram Blanche" sold by the company SPCI, "PUHP 3008" sold by the company Saint Gobain Ceramics;
- mica, or aluminosilicate, which may be chosen from muscovite, phlogogite, tiotite, sericite, lepidolite, paragonite, margarite, roscoelite, artificial or synthetic mica having a fluorine atom replacing the hydroxyl group of the natural mica, and the baked or calcined products of these micas. Micas generally exist in the form of scales having dimensions of 2 to 200 µm, preferably 5-70 µm and a thickness of 0.1 to 5 µm, preferably of 0.2-3 µm. As mica, it is possible for example to use those sold under the names "MICA SFG70" by the company ASPANGER, "MICA CONCORD 1000" by the company SCIAMA;
- and mixtures thereof.

**One of the lamellar fillers is boron nitride and is present in the composition in an amount ranging from 3 to 10% by weight, relative to the total weight of the composition, preferably from 5 to 8% by weight.**

According to a particular embodiment, the talc may be present in the composition according to the invention in an amount ranging from 5 to 55% by weight, relative to the total weight of the composition, preferably from 10 to 53% by weight.

According to a particular embodiment, the boron nitride may be present in the composition according to the invention in an amount ranging preferably **from 5 to 8% by weight,** relative to the total weight of the composition.

The lamellar fillers may be present in the composition according to the invention in an amount ranging from 20 to 70% by weight, even better from 25% to 50% by weight, and more preferably from 30% to 35% by weight.

The lamellar particle(s) may be present in the pulverulent phase in an amount ranging from 30 to 85% by weight, relative to the total weight of the composition, preferably from 40 to 75% by weight, and more preferably from 40 to 70% by weight.

According to a preferred embodiment, the lamellar particles and the pigments are present in the composition in an :amount such that the weight ratio of the lamellar particles to the pigments ranges from 0.5 to 5, preferably from 0.8 to 2.5.

### Additional particles

In addition to the lamellar particles described above, the composition according to the invention may comprise at least one additional non-lamellar particle of any shape, for example of spherical or oblong shape.

There may be mentioned spherical silica, polyamide (Nylon^{®}) powders, poly-β-alanine powders, polyethylene powders, polyurethane powders such as the hexamethylene diisocyanate and trimethylol hexyl lactone copolymer powder sold under the names PLASTIC POWDER D-400 by the company TOSHIKI, powders of tetrafluoroethylene polymers (Teflon^{®}), lauroyllysine, starch, boron nitride, polymeric hollow microspheres such as those of polyvinylidene chloride/acrylonitrile such as Expancel^{®} (Nobel Industrie), acrylic acid copolymers, silicone resin powders, in particular silsesquioxane powders (silicone resin powders in particular described in patent EP 293795; Tospearls^{®} from Toshiba, for example), particles of elastomeric polyorganosiloxanes, particles of polymethyl methacrylate, precipitated calcium carbonate, magnesium carbonate and hydrocarbonate, hydroxyapatite, hollow silica microspheres, glass or ceramic microcapsules, metallic soaps derived from organic carboxylic acids having from 8 to 22 carbon atoms, preferably 12 to 18 carbon atoms, for example zinc, magnesium or lithium stearate, zinc laurate, magnesium myristate, barium sulphate and mixtures thereof.

### Fatty binder

The composition according to the invention comprises at least one fatty binder.

The expression fatty binder for the purposes of the present application is understood to mean a fatty phase which generally comprises at least one oil. This type of fatty phase serves in particular as dispersing medium for the pulverulent phase.

Advantageously, the fatty binder may comprise at least one oil.

The oil may be chosen from the oils conventionally used as binder in loose or compact powders. These oils may be in particular chosen from:
- mink oil, turtle oil, soybean oil, grapeseed oil, sesame oil, corn oil, rapeseed oil, sunflower oil, cottonseed oil, avocado oil, olive oil, castor oil, jojoba oil, peanut oil;
- hydrocarbon oils, such as paraffin oils, squalane, petroleum jelly;
- fatty esters, such as isopropyl myristate, isopropyl palmitate, butyl stearate, isodecyl stearate, isocetyl stearate, hexyl laurate, isononyl isononanoate, 2-ethylhexyl palmitate, 2-hexyldecyl laurate, 2-octyldecyl palmitate, 2-octyldodecyl myristate or lactate, 2-diethylhexyl succinate, diisostearyl malate, glycerine or diglycerine triisostearate;
- silicone oils such as polymethylsiloxanes, polymethylphenylsiloxanes, polysiloxanes modified by fatty acids, fatty alcohols or polyoxyalkylenes, fluorinated silicones or perfluorinated oils;
- higher fatty acids such as myristic acid, palmitic acid, stearic acid, behenic acid, oleic acid, linoleic acid, linolenic acid or isostearic acid;
- higher fatty alcohols such as cetanol, stearyl alcohol or oleyl alcohol;
- polymethylfluoroalkyl dimethylsiloxanes of formula (I): 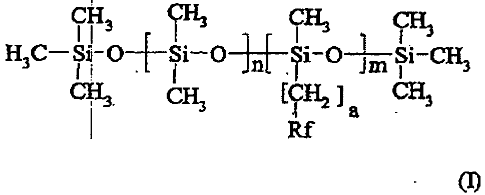 in which:
   - n represents an integer varying from 5 to 90, in particular from 30 to 80 and in particular from 50 to 80,
   - m represents an integer varying from 1 to 150, in particular from 1 to 80, and in particular from 1 to 40,
   - a represents an integer varying from 0 to 5, and
   - Rf denotes a perfluoroalkyl radical comprising from 1 to 8 carbon atoms; and
   - mixtures thereof.

According to a preferred embodiment, the oil is chosen from silicone oils such as polymethylsiloxanes, polymethylphenylsiloxanes, polysiloxanes modified with fatty acids, fatty alcohols or polyoxyalkylenes, fluorinated silicones or perfluorinated oils such as isopropyl myristate, isopropyl palmitate, butyl stearate, isodecyl stearate, isocetyl stearate, hexyl laurate, isononyl isononanoate, 2-ethylhexyl palmitate, 2-hexyldecyl laurate, 2-octyldecyl palmitate, 2-octyldodecyl myristate or lactate, 2-diethylhexyl succinate, diisostearyl malate, glycerine or diglycerine triisostearate.

The silicone oil may be chosen from linear silicone oils of low viscosity (ranging from 1 to 300 cSt).

Preferably, the silicone oil is linear polysiloxane consisting (except terminal groups) of units of formula (I) 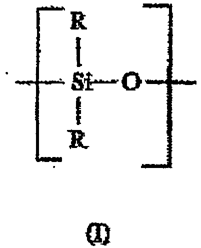 in which each substituent R independently represents a lower alkyl group (having 1 to 6 carbon atoms).

The degree of polymerization (number of repeating units) of these polysiloxanes of low viscosity may range for example from 3 to 2000 approximately.

These silicone oils of low viscosity may be prepared according to known methods, or bought from the market: there may be mentioned, for example, Silbione series 47 oil (RHONE POULENC), series 200 oil (DOW CORNING), SF 96 oil (GENERAL ELECTRIC) and mixtures thereof.

The terminal groups are, for example, trimethylsilyl, dimethyl hydroxymethylsilyl or vinyl dimethylsilyl groups.

The silicone oil may represent from 12 to 98.9% by weight relative to the total weight of the silicone binder.

The fatty ester oil may be isononyl isononanoate.

The oil may be present in the composition according to the invention in an amount ranging from 0.5 to 15% by weight, relative to the total weight of the composition, and in particular from 1 to 10% by weight, and more preferably from 1.5 to 5% by weight.

In addition to the oil, the fatty binder of the composition according to the invention may comprise a wax and/or silicone pasty fatty substances.

The waxes (and/or silicone pasty fatty substances) which can be used in the fatty binder of the composition according to the invention are substituted polysiloxanes, preferably of low melting point. They are in particular substituted linear polysiloxanes mainly consisting (apart from the terminal groups) of units of formulae II and III, in the respective molar proportions m and n: 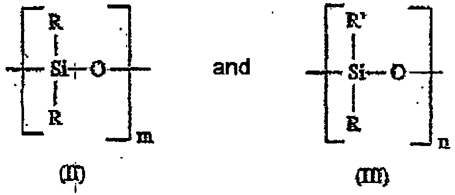 in which:
- each substituent R is as defined above,
- each R' independently represents an optionally unsaturated (linear or branched) alkyl having 6-30 carbon atoms, or alternatively a group -X-R", each X independently representing:

   -O-,

   -(CH2)a-O-CO-,

   -(CH2)b-CO-O-,

   a and b independently represent numbers which may vary from 0 to 6, and
   each R" independently represents an optionally unsaturated alkyl group having 6 to 30 carbon atoms,
- m is a number which may vary from 0 to 400, and in particular from 0 to 100,
- n is a number which may vary from 1 to 200, and in particular from 1 to 100,
the sum (m + n) being less than 400, and in particular less than or equal to 100.

These silicone waxes are known or may be prepared according to known methods. Among the commercial silicone waxes of Ithis type, there may be mentioned in particular those sold under the names Abilwax 9800, 9801 or 9810 (GOLDSCHMIDT), KF910 and KF7002 (SHIN ETSU), or 176-1118-3 and 176-11481 (GENERAL ELECTRIC).

The silicone waxes which can be used may also be chosen from the compounds of formula (IV):

R₁-Si(CH₃)₂-O-[Si(R)₂-O]₂-Si(CH₃)₂-R₂ (IV)

in which:
R is as defined above,
R₁ represents an alkyl group having from 1 to 30 carbon atoms, an alkoxyl group having from 6 to 30 carbon atoms, or a group of formula: 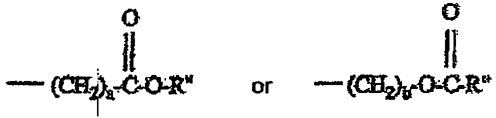
R₂ represents an alkyl group of 6 to 30 C, an alkoxy group having from 6 to 30 C or a group of formula: 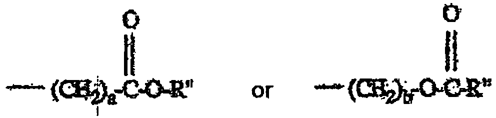 a and b representing a number from 0 to 6,
   R" being an alkyl having from 6 to 30 carbon atoms, and z is a number which may vary from 1 to 100.

Among the silicone waxes of formula (IV), which are known products or products which may be prepared according to known methods, there may be mentioned in particular the following commercial products: Abilwax 2428, 2434 and 2440 (GOLDSCHMIDT), or VP 1622 and VP 1621 (WHACKER).

The wax and/or the silicone pasty fatty substance may represent from 1 to 60% by weight relative to the total weight of the silicone binder.

In addition to the oil and/or the silicone wax, the fatty binder of the composition according to the invention may comprise a silicone resin.

Silicone resins are products of hydrolysis or of polycondensation of mixtures of siloxanes of formulae (R)₃SiOCH₃ and Si(OCH₃)₄, R representing an alkyl group having from 1 to 6 carbon atoms.

These silicone resins are known or may be prepared according to known methods. Among the commercial silicone resins which can be used, there may be mentioned for example those sold under the names DC 593 (DOW CORNING) or SS 4230 (GENERAL ELECTRIC).

The silicone resin may be present in an amount ranging from 0.1 to 25% by weight relative to the total weight of the silicone binder.

According to a preferred embodiment, the fatty binder of the composition according to the invention comprises at least two components chosen from:
(a) a silicone oil,
(b) a wax and/or a silicone pasty fatty substance,
(c) a silicone resin.

The fatty binder may be present in the composition in an amount ranging from 1 to 8% by weight, relative to the total weight of the composition, preferably from 2 to 7% by weight, more preferably from 3 to 6% by weight, and better still from 2 to 5% by weight.

### Additional wax

In addition to the wax which may be present in the fatty binder, the composition according to the invention may comprise at least one additional wax.

The expression "wax", for the purposes of the present invention, is understood to mean a lipophilic fatty compound which is solid at room temperature (25°C) and atmospheric pressure, (760 mmHg, that is 10⁵ Pa), which exhibits a reversible solid/liquid change of state, which has in particular a melting point greater than or equal to 30°C, in particular greater than or equal to 55°C, and which may be up to 250°C, in particular up to 230°C, and in particular up to 120°C.

By heating the wax to its melting point, it is possible to make it miscible with oils and to form a microscopically homogeneous mixture, but on allowing the temperature of the mixture to return to room temperature, recrystallization of the wax in the oils of the mixture is obtained.

The melting point values correspond, according to the invention, to the melting peak measured with the aid of differential scanning calorimetry (D.S.C.), for example the calorimeter sold under the name DSC 30 by the company METLER, with a temperature rise of 5 or 10°C per minute.

The waxes, for the purposes of the invention, may be those generally used in the cosmetic or dermatological fields. They may optionally be hydrocarbon-based, silicone-based and/or fluorinated, optionally comprising ester or hydroxyl functional groups. They may also be of natural or synthetic origin. By way of non-limiting illustration of these waxes, there may be mentioned in particular:
- beeswax, lanolin wax, and Chinese waxes; rice wax, Carnauba wax, Candelilla wax, Ouricury wax, cork fibre wax, sugar cane wax, Japan wax and sumac wax; montan wax, microcrystalline waxes, paraffin waxes, ozokerites, ceresin wax, lignite wax, polyethylene waxes, the waxes obtained by Fisher-Tropsch synthesis, fatty acid esters and glycerides which are concrete at 40°C and in particular at more than 55°C,
- waxes obtained by catalytic hydrogenation of animal or vegetable oils having linear or branched C₈-C₃₂ fatty chains, in particular hydrogenated jojoba oil, hydrogenated sunflower oil, hydrogenated castor oil, hydrogenated copra oil and hydrogenated lanolin oil,
- silicone waxes or fluorinated waxes, and
- mixtures thereof.

According to a particular variant of the invention, the solid fatty phase may comprise at least one wax chosen from carnauba waxes, paraffin waxes and mixtures thereof.

According to a preferred embodiment, the additional wax present in the composition according to the invention may be totally or partially in powdered, in particular micronized, form in order to facilitate its use in the preparation of the cosmetic composition.

Among the waxes which can be used in powdered form, there may be mentioned in particular the Carnauba wax microbeads sold under the name Microcare 350^{®} by the company Micro Powders and paraffin wax microbeads sold under the name Microease 114S^{®} by the company Micro Powders. Such additional micronized waxes make it possible in particular to improve the properties during the application of the composition to the skin.

The additional waxes may be present in the composition according to the invention in an amount ranging from 0.5 to 15% by weight, relative to the total weight of the composition, preferably from 0.8 to 5%, and more preferably from 0.9 to 1.2% by weight.

### Additives

The composition according to the invention may comprise at least one other customary cosmetic ingredient which may be chosen in particular from lipophilic gelling and/or thickening agents, antioxidants, perfumes, preservatives, neutralizing agents, sunscreens, vitamins, moisturizers, self-tanning compounds, antiwrinkle actives, emollients, hydrophilic or lipophilic actives, anti-pollution or anti-free radical agents, sequestrants, film-forming agents, non-elastomeric surfactants, skin-relaxing actives, soothing agents, agents stimulating the synthesis of skin or epidermal macromolecules and/or preventing their degradation, anti-glycation agents, anti-irritant agents, desquamating agents, depigmenting agents, anti-pigmenting agents or pro-pigmenting agents, NO-synthase inhibitors, agents stimulating the proliferation of fibroblasts or keranocytes and/or the differentiation of keranocytes, agents acting on the microcirculation, agents acting on the energy metabolism of the cells, wound-healing agents, and mixtures thereof.

According to a preferred embodiment, the composition according to the invention may be a cosmetic composition in powdered form, comprising:
i) 1 to 10% by weight, relative to the total weight of the composition, of at least one pigment chosen from iron oxides,
ii) 20 to 50% by weight, relative to the total weight of the composition, of pigments chosen from zinc oxides, titanium oxides and a mixture thereof,
iii) 25 to 45% by weight, relative to the total weight of the composition, of at least one lamellar pearlescent agent,
iv) 15 to 35 by weight, relative to the total weight of the composition, of at least one lamellar filler,
v) 2 to 7% by weight, relative to the total weight of the composition, of at least one fatty binder.

According to a preferred embodiment, the composition according to the invention may be an anhydrous composition. The expression anhydrous composition is understood to mean a composition containing less than 2% by weight of water, or even less than 0.5% of water, and in particular free of water, water not being added during the preparation of the composition but corresponding to the residual water provided by the ingredients mixed.

The invention is illustrated in greater detail in the examples below.

### Examples 1 and 2: Loose powders

Two loose powders having the following compositions were prepared:

| | MP Name | Ex. 1 (% by weight) | Ex. 2 (% by weight) | Ex. 3 (% by weight) |
|---|---|---|---|---|
| Phase A1 | Yellow iron oxide | 4.0 | 13.4 | 6.00 |
| | Red iron oxide | 1.3 | 7.6 | 1.7 |
| | Black iron oxide | 0.4 | 4.0 | 0.7 |
| | Bismuth oxychloride | 30.0 | 5.0 | 36.5 |
| | Titanium dioxide | 20.0 | 3.0 | 20.0 |
| | Zinc!oxide | 20.0 | 5.0 | - |
| | Boron nitride | 6.0 | 6.0 | 6.0 |
| | Carnauba wax | 1.0 | 1.0 | 1.0 |
| | Mica | | | 20.9 |
| | Talc | 13.7 | 51.4 | - |
| Phase A2 | Silicone binder | 3.0 | 3.0 | |
| | Hydrocarbon binder | | | 2.00 |
| | Vitamin B5 | | | qs |
| | Aloe extract | | | qs |
| | Vitamin E | | | qs |
| | Preservative | 0.6 | 0.6 | |

All the constituents of phase A1 are mixed in a Lodige mixer for 15 minutes (without emptying).

The constituents of phase A2 are added, with stirring, to A1 and the mixture is mixed for 15 minutes.

The mixture obtained is then ground in a pin mill at a speed of 1800 revolutions/min.

The mixture obtained is sieved on a 400 micron sieve.

The loose powder obtained has good properties of softness during its application to the skin. After application, the make-up makes it possible to effectively conceal the colour defects of the skin, while conferring a natural and satiny appearance on the made-up skin.

## Claims

1. Anhydrous cosmetic composition in loose powdered form comprising at least one pulverulent phase and a fatty binder, the said pulverulent phase comprising:
i) 15 to 70% by weight, relative to the total weight of the composition, of at least one pigment, and,
ii) 30 to 85% by weight, relative to the total weight of the composition, **of at least one lamellar particle other than the pigment, the pulverulent phase comprising at** least one lamellar pearlescent agent and at least two lamellar fillers chosen from talc, mica, boron nitride, kaolin and a mixture thereof, wherein one of the lamellar fillers is boron nitride being present in the composition in an amount ranging from 3 to 10% by weight, relative to the total weight of the composition, preferably from 5 to 8% by weight.

2. Composition according to claim 1, **characterized in that** the lamellar pearlescent agent is chosen from white pearlescent agents such as mica coated with titanium or with bismuth oxychloride, coloured pearlescent agents such as mica-titanium coated with iron oxides, mica-titanium coated with in particular ferric blue or chromium oxide, mica-titanium coated with an organic pigment of the abovementioned type and pearlescent agents made of bismuth oxychloride or based on bismuth oxychloride.

3. Composition according to either claim 1 or 2, **characterized in that** the lamellar pearlescent agent is bismuth oxychloride.

4. Composition according to any one of Claims 1 to 3, **characterized in that** the lamellar pearlescent agent is present in the composition in an amount ranging from 3 to 70% by weight, relative to the total weight of the composition, preferably from 5 to 40% by weight.

5. Composition according to claim 1, **characterized in that** the talc is present in the composition in an amount ranging from 5 to 55% by weight, relative to the total weight of the composition, preferably from 10 to 53% by weight.

6. Composition according to claim 1, **characterized in that** the lamellare fillers are present in the composition in an amount ranging from 20 to 70% by weight, even better from 25% to 50%, and more preferably from 30% to 35% by weight.

7. Composition according to any one of the preceding claims, **characterized in that** the lamellar particles are present in the pulverulent phase in an amount ranging from 30 to 85% by weight, relative to the total weight of the composition, preferably from 40 to 75% by weight, and more preferably from 40 to 70% by weight.

8. Composition according to any one of the preceding claims, **characterized in that** the lamellar particles and the pigments are present in the composition in an amount such that the weight ratio of lamellar particles to pigments ranges from 0.5 to 5, preferably from 0.8 to 2.5.

9. Composition according to the claim 1, **characterized in that** the pigment is chosen from pigments of metal oxides.

10. Composition according to the preceding claim, **characterized in that** the pigments of metal oxides are chosen from pigments of iron oxide, zinc oxide and/or titanium dioxide.

11. Composition according to any one of the preceding claims, **characterized in that** the pulverulent phase comprises at least two different pigments.

12. Composition according to any one of the preceding claims, **characterized in that** the pulverulent phase comprises at least a first pigment chosen from pigments of iron oxide and at least a second pigment chosen from pigments of zinc oxide and/or titanium dioxide.

13. Composition according to any one of the preceding claims, **characterized in that** the pigments are present in the composition in an amount ranging from 15 to 70% by weight, relative to the total weight of the composition, preferably from 25 to 70% by weight, relative to the total weight of the composition, preferably from 26 to 60% by weight, and more preferably from 27 to 50% by weight.

14. Composition according to either of Claims 12 and 13, **characterized in that** the pigments of iron oxide are present in the composition in an amount ranging from 1 to 40% by weight, relative to the total weight of the composition, preferably from 3 to 30% by weight, and more preferably from 4 to 25% by weight.

15. Composition according to either of Claims 12 and 13, **characterized in that** the pigments of zinc oxide are present in the composition in an amount ranging from 5 to 55% by weight, relative to the total weight of the composition, preferably from 10 to 40% by weight, and more preferably from 15 to 25% by weight.

16. Composition according to either of Claims 12 and 13, **characterized in that** the pigments of titanium dioxide are present in the composition in an amount ranging from 3 to 55% by weight, relative to the total weight of the composition, preferably from 10 to 40% by weight, and more preferably from 15 to 25% by weight.

17. Composition according to any one of Claims 12 to 13 and 15 to 16, **characterized in that** the pigments of zinc oxide and the pigments of titanium dioxide are present in the composition in an amount such that the weight ratio of zinc oxide to titanium dioxide ranges from 0.5 to 2, preferably from 0.8 to 1.8, and more preferably still from 0.9 to 1.7.

18. Composition according to any one of Claims 12 to 17, **characterized in that** the pigments of iron oxide and zinc oxide and the pigments of titanium dioxide are present in the composition in an amount such that the weight ratio of the sum of the zinc oxides and/or titanium dioxides to the iron oxides ranges from 0.1 to 10 and preferably from 0.2 to 8.

19. Composition according to any one of the preceding claims, **characterized in that** the fatty binder comprises an oil.

20. Composition according to the preceding claim, **characterized in that** the oil is chosen from silicone oils such as polymethylsiloxanes, polymethylphenylsiloxanes, polysiloxanes modified with fatty acids, fatty alcohols or polyoxyalkylenes, fluorinated silicones or perfluorinated oils and fatty ester oils such as isopropyl myristate, isopropyl palmitate, butyl stearate, isodecyl stearate, isocetyl stearate, hexyl laurate, isononyl isononanoate, 2-ethylhexyl palmitate, 2-hexyldecyl laurate, 2-octyldecyl palmitate, 2-octyldodecyl myristate or lactate, 2-diethylhexyl succinate, diisostearyl malate, glycerine or diglycerine triisostearate.

21. Composition according to any one of the preceding claims, **characterized in that** the fatty binder comprises a wax! and/or a silicone pasty fatty substance.

22. Composition according to any one of the preceding claims, **characterized in that** the fatty binder comprises a silicone resin.

23. Composition according to any one of the preceding claims, **characterized in that** it comprises at least one additional wax.

24. Composition according to the preceding claim, **characterized in that** the additional wax is chosen from:
- beeswax, lanolin wax, and Chinese waxes; rice wax, Carnauba wax, Candelilla wax, Ouricury wax, cork fibre wax, sugar cane wax, Japan wax and sumac wax; montan wax, microcrystalline waxes, paraffin waxes, ozokerites, ceresin wax, lignite wax, polyethylene waxes, the waxes obtained by Fisher-Tropsch synthesis, fatty acid esters and glycerides which are concrete at 40°C and in particular at more than 55°C,
- waxes obtained by catalytic hydrogenation of animal or vegetable oils having linear or branched C₈-C₃₂ fatty chains, in particular hydrogenated jojoba oil, hydrogenated sunflower oil, hydrogenated castor oil, hydrogenated copra oil and hydrogenated lanolin oil,
- silicone waxes or fluorinated waxes,
- zinc laurate, magnesium stearate, magnesium myristate, zinc stearate, and
- mixtures thereof.

25. Composition according to the preceding claim, **characterized in that** the additional wax is totally or partially in powdered form.

26. Composition according to the preceding claim, **characterized in that** the wax is chosen from carnauba waxes, paraffin waxes and mixtures thereof.

27. Composition according to any one of Claims 23 to 26, **characterized in that** the additional wax is present in an amount ranging from 0.5 to 15% by weight, relative to the total weight of the composition, in particular from 0.8 to 5% by weight, and in particular from 0.9 to 1.2% by weight.

28. Method for making up the skin of the face and/or of the body, comprising the application, to the said skin, of a composition according to any one of Claims 1 to 27.

29. Use of a composition according to any one of Claims 1 to 27, for obtaining a make-up capable of concealing the colour defects of the skin, while conferring a natural and satiny appearance on the made-up skin.

## Patentansprüche

1. Wasserfreie kosmetische Zusammensetzung in lose gepulverter Form, umfassend wenigstens eine feinpulverige Phase und ein fettartiges Bindemittel, wobei die feinpulverige Phase umfasst:
i) 15 bis 70 Gew.-% bezogen auf das Gesamtgewicht der Zusammensetzung an wenigstens einem Pigment und
ii) 30 bis 85 Gew.-% bezogen auf das Gesamtgewicht der Zusammensetzung an wenigstens einem lamellaren Partikel, das von dem Pigment verschieden ist, wobei die feinpulverige Phase wenigstens ein lamellares Perlglanzmittel und wenigstens zwei lamellare Füllstoffe ausgewählt aus Talkum, Glimmer, Bornitrid, Kaolin und einem Gemisch davon umfasst, wobei einer der lamellaren Füllstoffe Bornitrid ist, das in der Zusammensetzung in einer Menge in dem Bereich von 3 bis 10 Gew.-% bezogen auf das Gesamtgewicht der Zusammensetzung vorhanden ist, vorzugsweise von 5 bis 8 Gew.-%.

2. Zusammensetzung gemäß Anspruch 1, **dadurch gekennzeichnet, dass** das lamellare Perlglanzmittel ausgewählt ist aus weißen Perlglanzmitteln, wie z. B. Glimmer überzogen mit Titan oder mit Bismutoxychlorid, gefärbten Perlglanzmitteln, wie z. B. Glimmer-Titan überzogen mit Eisenoxiden, Glimmer-Titan überzogen mit insbesondere Eisenblau oder Chromoxid, Glimmer-Titan überzogen mit einem organischen Pigment des vorstehend genannten Typs, und Perlglanzmitteln, die aus Bismutoxychlorid bestehen oder auf Bismutoxychlorid basieren.

3. Zusammensetzung gemäß Anspruch 1 oder 2, **dadurch gekennzeichnet, dass** das lamellare Perlglanzmittel Bismutoxychlorid ist.

4. Zusammensetzung gemäß einem der Ansprüche 1 bis 3, **dadurch gekennzeichnet, dass** das lamellare Perlglanzmittel in der Zusammensetzung in einer Menge in dem Bereich von 3 bis 70 Gew.-% bezogen auf das Gesamtgewicht der Zusammensetzung vorhanden ist, vorzugsweise von 5 bis 40 Gew.-%.

5. Zusammensetzung gemäß Anspruch 1, **dadurch gekennzeichnet, dass** das Talkum in der Zusammensetzung in einer Menge in dem Bereich von 5 bis 55 Gew.-% bezogen auf das Gesamtgewicht der Zusammensetzung vorhanden ist, vorzugsweise von 10 bis 53 Gew.-%.

6. Zusammensetzung gemäß Anspruch 1, **dadurch gekennzeichnet, dass** die lamellaren Füllstoffe in der Zusammensetzung in einer Menge in dem Bereich von 20 bis 70 Gew.-% vorhanden sind, noch besser von 25 Gew.-% bis 50 Gew.-% und bevorzugter von 30 Gew.-% bis 35 Gew.-%.

7. Zusammensetzung gemäß einem der vorstehenden Ansprüche, **dadurch gekennzeichnet, dass** die lamellaren Partikel in der feinpulverigen Phase in einer Menge in dem Bereich von 30 bis 85 Gew.-% bezogen auf das Gesamtgewicht der Zusammensetzung vorhanden sind, vorzugsweise von 40 bis 75 Gew.-% und bevorzugter von 40 bis 70 Gew.-%.

8. Zusammensetzung gemäß einem der vorstehenden Ansprüche, **dadurch gekennzeichnet, dass** die lamellaren Partikel und die Pigmente in der Zusammensetzung in einer solchen Menge vorhanden sind, dass das Gewichtsverhältnis von lamellaren Partikeln zu Pigmenten in dem Bereich von 0,5 bis 5 liegt, vorzugsweise von 0,8 bis 2,5.

9. Zusammensetzung gemäß Anspruch 1, **dadurch gekennzeichnet, dass** das Pigment ausgewählt ist aus Pigmenten von Metalloxiden.

10. Zusammensetzung gemäß dem vorstehenden Anspruch, **dadurch gekennzeichnet, dass** die Pigmente von Metalloxiden ausgewählt sind aus Pigmenten von Eisenoxid, Zinkoxid und/oder Titandioxid.

11. Zusammensetzung gemäß einem der vorstehenden Ansprüche, **dadurch gekennzeichnet, dass** die feinpulverige Phase wenigstens zwei verschiedene Pigmente umfasst.

12. Zusammensetzung gemäß einem der vorstehenden Ansprüche, **dadurch gekennzeichnet, dass** die feinpulverige Phase wenigstens ein erstes Pigment ausgewählt aus Pigmenten von Eisenoxid und wenigstens ein zweites Pigment ausgewählt aus Pigmenten von Zinkoxid und/oder Titandioxid umfasst.

13. Zusammensetzung gemäß einem der vorstehenden Ansprüche, **dadurch gekennzeichnet, dass** die Pigmente in der Zusammensetzung in einer Menge in dem Bereich von 15 bis 70 Gew.-% bezogen auf das Gesamtgewicht der Zusammensetzung vorhanden sind, vorzugsweise von 25 bis 70 Gew.-% bezogen auf das Gesamtgewicht der Zusammensetzung, vorzugsweise von 26 bis 60 Gew.-% und bevorzugter von 27 bis 50 Gew.-%.

14. Zusammensetzung gemäß einem der Ansprüche 12 und 13, **dadurch gekennzeichnet, dass** die Pigmente von Eisenoxid in der Zusammensetzung in einer Menge in dem Bereich von 1 bis 40 Gew.-% bezogen auf das Gesamtgewicht der Zusammensetzung vorhanden sind, vorzugsweise von 3 bis 30 Gew.-% und bevorzugter von 4 bis 25 Gew.-%.

15. Zusammensetzung gemäß einem der Ansprüche 12 und 13, **dadurch gekennzeichnet, dass** die Pigmente von Zinkoxid in der Zusammensetzung in einer Menge in dem Bereich von 5 bis 55 Gew.-% bezogen auf das Gesamtgewicht der Zusammensetzung vorhanden sind, vorzugsweise von 10 bis 40 Gew.-% und bevorzugter von 15 bis 25 Gew.-%.

16. Zusammensetzung gemäß einem der Ansprüche 12 und 13, **dadurch gekennzeichnet, dass** die Pigmente von Titandioxid in der Zusammensetzung in einer Menge in dem Bereich von 3 bis 55 Gew.-% bezogen auf das Gesamtgewicht der Zusammensetzung vorhanden sind, vorzugsweise von 10 bis 40 Gew.-% und bevorzugter von 15 bis 25 Gew.-%.

17. Zusammensetzung gemäß einem der Ansprüche 12 bis 13 und 15 bis 16, **dadurch gekennzeichnet, dass** die Pigmente von Zinkoxid und die Pigmente von Titandioxid in der Zusammensetzung in einer solchen Menge vorhanden sind, dass das Gewichtsverhältnis von Zinkoxid zu Titandioxid in dem Bereich von 0,5 bis 2 liegt, vorzugsweise von 0,8 bis 1,8 und noch bevorzugter von 0,9 bis 1,7.

18. Zusammensetzung gemäß einem der Ansprüche 12 bis 17, **dadurch gekennzeichnet, dass** die Pigmente von Eisenoxid und Zinkoxid und die Pigmente von Titandioxid in der Zusammensetzung in einer solchen Menge vorhanden sind, dass das Gewichtsverhältnis der Summe der Zinkoxide und/oder Titandioxide zu den Eisenoxiden in dem Bereich von 0,1 bis 10 liegt, vorzugsweise von 0,2 bis 8.

19. Zusammensetzung gemäß einem der vorstehenden Ansprüche, **dadurch gekennzeichnet, dass** das fettartige Bindemittel ein Öl umfasst.

20. Zusammensetzung gemäß dem vorstehenden Anspruch, **dadurch gekennzeichnet, dass** das Öl ausgewählt ist aus Siliconölen, wie z. B. Polymethylsiloxanen, Polymethylphenylsiloxanen, Polysiloxanen modifiziert mit Fettsäuren, Fettalkoholen oder Polyoxyalkylenen, fluorierten Siliconen oder perfluorierten Ölen und Fettsäureesterölen, wie z. B. Isopropylmyristat, Isopropylpalmitat, Butylstearat, Isodecylstearat, Isocetylstearat, Hexyllaurat, Isononylisononanoat, 2-Ethylhexylpalmitat, 2-Hexyldecyllaurat, 2-Octyldecylpalmitat, 2-Octyldodecylmyristat oder -lactat, 2-Diethylhexylsuccinat, Diisostearylmalat, Glycerin- oder Diglycerintriisostearat.

21. Zusammensetzung gemäß einem der vorstehenden Ansprüche, **dadurch gekennzeichnet, dass** das fettartige Bindemittel ein Wachs und/oder einen fettartigen Siliconstoff umfasst.

22. Zusammensetzung gemäß einem der vorstehenden Ansprüche, **dadurch gekennzeichnet, dass** das fettartige Bindemittel ein Siliconharz umfasst.

23. Zusammensetzung gemäß einem der vorstehenden Ansprüche, **dadurch gekennzeichnet, dass** sie wenigstens ein zusätzliches Wachs umfasst.

24. Zusammensetzung gemäß dem vorstehenden Anspruch, **dadurch gekennzeichnet, dass** das zusätzliche Wachs ausgewählt ist aus:
- Bienenwachs, Lanolinwachs und Chinawachsen; Reiswachs, Carnaubawachs, Candelillawachs, Ouricurywachs, Korkfaserwachs, Zuckerrohrwachs, Japanwachs und Sumacwachs; Montanwachs, mikrokristallinen Wachsen, Paraffinwachsen, Ozokeriten, Ceresinwachs, Lignitwachs, Polyethylenwachsen, den durch Fischer-Tropsch-Synthese erhaltenen Wachsen, Fettsäureestern und Glyceriden, die bei 40 °C, insbesondere bei über 55 °C, konkret sind,
- Wachsen, die durch katalytische Hydrierung von Tier- oder Pflanzenölen mit linearen oder verzweigten C₈-C₃₂-Fettsäureketten erhalten sind, insbesondere hydriertes Jojobaöl, hydriertes Sonnenblumenöl, hydriertes Castoröl, hydriertes Copraöl und hydriertes Lanolinöl,
- Siliconwachsen oder fluorierten Wachsen,
- Zinklaurat, Magnesiumstearat, Magnesiummyristat, Zinkstearat und
- Gemischen davon.

25. Zusammensetzung gemäß dem vorstehenden Anspruch, **dadurch gekennzeichnet, dass** das zusätzliche Wachs vollständig oder teilweise in pulverförmiger Form vorliegt.

26. Zusammensetzung gemäß dem vorstehenden Anspruch, **dadurch gekennzeichnet, dass** das Wachs ausgewählt ist aus Carnaubawachsen, Paraffinwachsen und Gemischen davon.

27. Zusammensetzung gemäß einem der Ansprüche 23 bis 26, **dadurch gekennzeichnet, dass** das zusätzliche Wachs in einer Menge in dem Bereich von 0,5 bis 15 Gew.-% bezogen auf das Gesamtgewicht der Zusammensetzung vorhanden ist, insbesondere von 0,8 bis 5 Gew.-% und insbesondere von 0,9 bis 1,2 Gew.-%.

28. Verfahren zum Make-up der Haut des Gesichts und/oder des Körpers, umfassend das Aufbringen einer Zusammensetzung gemäß einem der Ansprüche 1 bis 27 auf die Haut.

29. Verwendung einer Zusammensetzung gemäß einem der Ansprüche 1 bis 27 zum Erhalten eines Make-ups, das fähig ist, die Farbdefekte der Haut zu verbergen, während es der Make-up-behandelten Haut ein natürliches und seidiges Aussehen verleiht.

## Revendications

1. Composition cosmétique anhydre sous forme de poudre libre comprenant au moins une phase pulvérulente et un liant gras, ladite phase pulvérulente comprenant :
i) 15 à 70 % en poids, par rapport au poids total de la composition, d'au moins un pigment, et
ii) 30 à 85 % en poids, par rapport au poids total de la composition, d'au moins une particule lamellaire autre que le pigment, la phase pulvérulente comprenant au moins une nacre lamellaire et au moins deux charges lamellaires choisies parmi le talc, le mica, le nitrure de bore, le kaolin et un mélange de ceux-ci, l'une des charges lamellaires étant le nitrure de bore qui est présent dans la composition en une teneur allant de 3 à 10 % en poids, par rapport au poids total de la composition, de préférence de 5 à 8 % en poids.

2. Composition selon la revendication 1, **caractérisée en ce que** la nacre lamellaire est choisie parmi les nacres blanches telles que le mica recouvert de titane ou d'oxychlorure de bismuth, les nacres colorées tels que le mica titane recouvert avec des oxydes de fer, le mica titane recouvert avec notamment du bleu ferrique ou de l'oxyde de chrome, le mica titane recouvert avec un pigment organique du type précité ainsi que les nacres d'oxychlorure de bismuth ou à base d'oxychlorure de bismuth.

3. Composition selon l'une quelconque des revendications 1 et 2, **caractérisée en ce que** la nacre lamellaire est l'oxychlorure de bismuth.

4. Composition selon l'une quelconque des revendications 1 à 3, **caractérisée en ce que** la nacre lamellaire est présente dans la composition en une teneur allant de 3 à 70 % en poids, par rapport au poids total de la composition, de préférence de 5 à 40 % en poids.

5. Composition selon la revendication 1, **caractérisée en ce que** le talc est présent dans la composition en une teneur allant de 5 à 55 % en poids, par rapport au poids total de la composition, de préférence de 10 à 53 % en poids.

6. Composition selon la revendication 1, **caractérisée en ce que** les charges lamellaires sont présentes dans la composition en une teneur allant de 20 à 70 % en poids, mieux de 25 % à 50 % en poids, et plus préférentiellement de 30 % à 35 % en poids.

7. Composition selon l'une quelconque des revendications précédentes, **caractérisée en ce que** les particules lamellaires sont présentes dans la phase pulvérulente en une teneur allant de 30 à 85 % en poids, par rapport au poids total de la composition, de préférence de 40 à 75 % en poids, et plus préférentiellement de 40 à 70 % en poids.

8. Composition selon l'une quelconque des revendications précédentes, **caractérisée en ce que** les particules lamellaires et les pigments sont présents dans la composition en une teneur telle que le rapport pondéral particules lamellaires sur pigments va de 0,5 à 5, de préférence de 0,8 à 2,5.

9. Composition selon la revendication 1, **caractérisée en ce que** le pigment est choisi parmi des pigments d'oxydes métalliques.

10. Composition selon la revendication précédente, **caractérisée en ce que** les pigments d'oxydes métalliques sont choisis parmi les pigments d'oxyde de fer, d'oxyde de zinc, et/ou de dioxyde de titane.

11. Composition selon l'une quelconque des revendications précédentes, **caractérisée en ce que** phase pulvérulente comprend au moins deux pigments différents.

12. Composition selon l'une quelconque des revendications précédentes, **caractérisée en ce que** phase pulvérulente comprend au moins un premier pigment choisi parmi les pigments d'oxyde de fer et au moins un second pigment choisi parmi les pigments d'oxyde de zinc et/ou de dioxyde de titane.

13. Composition selon l'une quelconque des revendications précédentes, **caractérisée en ce que** les pigments sont présents dans la composition en une teneur allant de 15 à 70 % en poids, par rapport au poids total de la composition, de préférence de 25 à 70 % en poids, par rapport au poids total de la composition, de préférence de 26 à 60 % en poids, et plus préférentiellement de 27 à 50 % en poids.

14. Composition selon l'une quelconque des revendications 12 et 13, **caractérisée en ce que** les pigments d'oxydes de fer sont présents dans la composition en une teneur allant de 1 à 40 % en poids, par rapport au poids total de la composition, de préférence de 3 à 30 % en poids, et plus préférentiellement de 4 à 25 % en poids.

15. Composition selon l'une quelconque des revendications 12 et 13, **caractérisée en ce que** les pigments d'oxyde de zinc sont présents dans la composition en une teneur allant de 5 à 55 % en poids, par rapport au poids total de la composition, de préférence de 10 à 40 % en poids, et plus préférentiellement de 15 à 25 % en poids.

16. Composition selon l'une quelconque des revendications 12 et 13, **caractérisée en ce que** les pigments de dioxyde de titane sont présents dans la composition en une teneur allant de 3 à 55 % en poids, par rapport au poids total de la composition, de préférence de 10 à 40 % en poids, et plus préférentiellement de 15 à 25 % en poids.

17. Composition selon l'une quelconque des revendications 12 et 13 et 15 et 16, **caractérisée en ce que** les pigments d'oxyde de zinc et les pigments de dioxyde de titane sont présents dans la composition en une teneur telle que le rapport pondéral oxyde de zinc sur dioxyde de titane va de 0,5 à 2, de préférence de 0,8 à 1,8 et plus préférentiellement encore de 0,9 à 1,7.

18. Composition selon l'une quelconque des revendications 12 à 17, **caractérisée en ce que** les pigments d'oxyde de fer et d'oxyde de zinc et les pigments de dioxyde de titane sont présents dans la composition en une teneur telle que le rapport pondéral de la somme des oxydes de zinc et/ou des dioxydes de titane sur les oxydes de fer va de 0,1 à 10, de préférence de 0,2 à 8.

19. Composition selon l'une quelconque des revendications précédentes, **caractérisée en ce que** le liant gras comprend une huile.

20. Composition selon la revendication précédente, **caractérisée en ce que** l'huile est choisie parmi les huiles de silicone telles que les polyméthylsiloxanes, les polyméthylphénylsiloxanes, les polysiloxanes modifiés par des acides gras, des alcools gras ou des polyoxyalkylènes, les silicones fluorées ou les huiles perfluorées et les huiles d'esters gras, tels que le myristate d'isopropyle, le palmitate d'isopropyle, le stéarate de butyle, le stéarate d'isodécyle, le stéarate d'isocétyle, le laurate d'hexyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthyl-hexyle, le malate de diisostéaryle, le triisostéarate de glycérine ou de diglycérine.

21. Composition selon l'une quelconque des revendications précédentes, **caractérisée en ce que** le liant gras comprend une cire et/ou un corps gras pâteux de silicone.

22. Composition selon l'une quelconque des revendications précédentes, **caractérisée en ce que** le liant gras comprend une résine de silicone.

23. Composition selon l'une quelconque des revendications précédentes, **caractérisée en ce qu'**elle comprend au moins une cire additionnelle.

24. Composition selon la revendication précédente, **caractérisée en ce que** la cire additionnelle est choisie parmi :
- la cire d'abeilles, la cire de lanoline, et les cires d'insectes de Chine ; la cire de riz, la cire de carnauba, la cire de candellila, la cire d'ouricury, la cire de fibres de liège, la cire de canne à sucre, la cire du Japon et la cire de sumac ; la cire de montan, les cires microcristallines, les cires de paraffine, les ozokérites, la cire de cérésine, la cire de lignite, les cires de polyéthylène, les cires obtenues par la synthèse de Fisher-Tropsch, les esters d'acides gras et les glycérides concrets à 40 °C et notamment à plus de 55 °C,
- les cires obtenues par hydrogénation catalytique d'huiles animales ou végétales ayant des chaînes grasses, linéaires ou ramifiées, en C₈-C₃₂, notamment l'huile de jojoba hydrogénée, l'huile de tournesol hydrogénée, l'huile de ricin hydrogénée, l'huile de coprah hydrogénée et l'huile de lanoline hydrogénée,
- les cires de silicone ou les cires fluorées,
- le laurate de zinc, le stéarate de magnésium, le myristate de magnésium, le stéarate de zinc, et
- leurs mélanges.

25. Composition selon la revendication précédente, **caractérisée en ce que** la cire additionnelle est totalement ou partiellement sous forme de poudre.

26. Composition selon la revendication précédente, **caractérisée en ce que** la cire est choisie parmi les cires de carnauba, les cires de paraffine et leurs mélanges.

27. Composition selon l'une quelconque des revendications 23 à 26, **caractérisée en ce que** la cire additionnelle est présente en une teneur allant de 0,5 à 15 % en poids, par rapport au poids total de la composition, notamment de 0,8 à 5 % en poids, et en particulier de 0,9 à 1,2 % en poids.

28. Procédé de maquillage de la peau du visage et/ou du corps comprenant l'application sur ladite peau d'une composition selon l'une quelconque des revendications 1 à 27.

29. Utilisation d'une composition selon l'une quelconque des revendications 1 à 27 pour obtenir un maquillage apte à camoufler les défauts de couleur de la peau, tout en conférant à la peau maquillée un aspect naturel et satiné.
